(19) Europäisches Patentamt
European Patent Office
Office européen des brevets

(11) Publication number: **0 103 366**
**B1**

(12) **EUROPEAN PATENT SPECIFICATION**

(45) Date of publication of patent specification: **19.02.86**

(51) Int. Cl.$^4$: **C 07 D 405/14**

(21) Application number: **83303899.5**

(22) Date of filing: **05.07.83**

(54) **A process for the preparation of 2-(2-((5-dimethylaminomethyl)furan-2-ylmethylthio)ethylamino)-5-(6-methylpyrid-3-ylmethyl)pyrimid-4-one.**

(30) Priority: **20.07.82 GB 8220898**

(43) Date of publication of application:
**21.03.84 Bulletin 84/12**

(45) Publication of the grant of the patent:
**19.02.86 Bulletin 86/08**

(84) Designated Contracting States:
**AT BE CH DE FR GB IT LI LU NL SE**

(56) References cited:
**EP-A-0 003 677**

**CHEMICAL ABSTRACTS, vol. 96, no. 19, May 10, 1982, Columbus, Ohio, USA DAMANN "Inhibition of nightly secretion" page 61, column 2, abstract no. 155 320k**

(73) Proprietor: **SMITH KLINE & FRENCH LABORATORIES LIMITED**
**Mundells**
**Welwyn Garden City Hertfordshire, AL7 1EY (GB)**

(72) Inventor: **Cooper, John**
**2 Waverley Drive Sandown Park**
**Tunbridge Wells Kent (GB)**
Inventor: **Forth, Michael Anthony**
**29 Fell Mead**
**Eact Peckham Near Tonbridge Kent (GB)**
Inventor: **Tremper, Alan William**
**329 Owen Avenue**
**Lansdown, Pennsylvania 19026 (US)**
Inventor: **Wert, Kathleen Laura**
**5521 Morris Street**
**Philadelphia Pennsylvania 19144 (US)**

(74) Representative: **Waters, David Martin, Dr. et al**
**Smith Kline & French Laboratories Ltd. Patent Department Mundells**
**Welwyn Garden City Hertfordshire AL7 1EY (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 103 366 B1**

Courier Press, Leamington Spa, England.

**Description**

The present invention relates to a process for the preparation of Mannich bases which have activity as histamine $H_2$-antagonists.

European Patent Application Publication No 0003677 discloses inter alia a process for the preparation of compounds of the formula (I):—

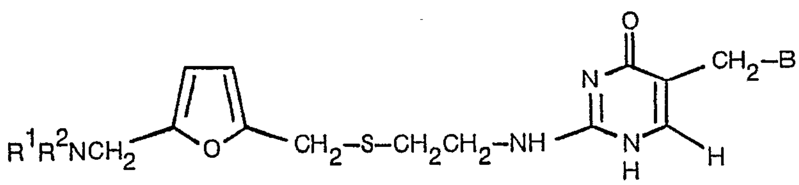

(I)

wherein $R^1$ and $R^2$ are lower alkyl or $R^1$ and $R^2$ together with the nitrogen atom to which they are attached form a pyrrolidino or piperidino group, and B is 3-pyridyl, 6-methyl-3-pyridyl, 5,6-dimethyl-3-pyridyl, 6-methoxy-3-pyridyl, 2-methoxy-4-pyridyl, or 6-hydroxy-3-pyridyl 2-hydroxy-4-pyridyl; which process comprises the reaction of a compound of the formula (II):—

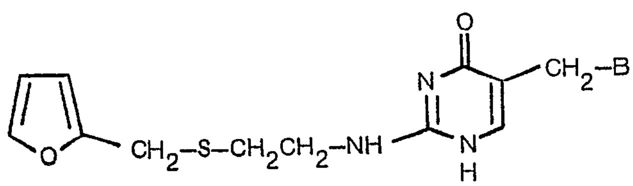

(II)

wherein B is as defined in relation to formula (I), with a Mannich reagent.

The compound of the formula (I) wherein B is 6-methyl-pyrid-3-ylmethyl and $R^1$ and $R^2$ are both methyl is one of considerable interest in the literature, an example of a literature reference being Dammann et al., Chemical Abstracts, vol 96, 155320k, 1982.

We have found that when the compound, 2-[2-[furan-2-ylmethylthio)ethylamino]-5-(6-methylpyrid-3-ylmethyl)-pyrimid-4-one, of the formula (III) is reacted with the Mannich reagent bis(dimethylamino)methane, the purity of the product is sensitive to the conditions under which the process is carried out. The formation of the desired product of the formula (IV), under acidic conditions, is accompanied by 2—3% of a by-product of the formula (V), named for convenience as 'the bis-Mannich compound'; see Scheme I.

SCHEME I

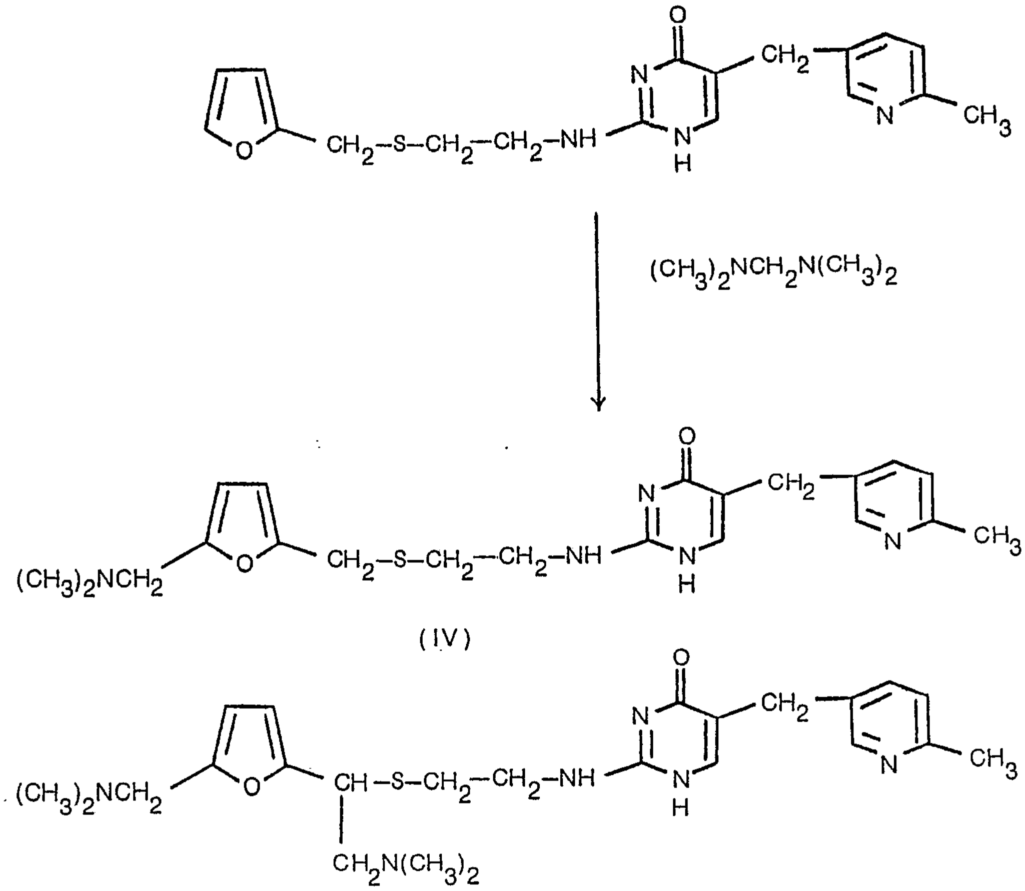


(IV)

(V)

'bis-Mannich Compound'

The compounds of the formulae (IV) and (V) have chemical structures and properties that make separation by conventional commercially feasible techniques difficult. However dissolving the product mixture in a biphase water organic solvent system and adjusting the pH of the aqueous layer to the range 6.7 to 6.9 affords better purification than other methods tried. However, during this purification procedure, we discovered that the compound of the formula (IV) dissoved in a chlorinated organic solvent, for example dichloromethane, at about neutral pH forms a substantial amount of a quaternary salt of the formula (VI):—

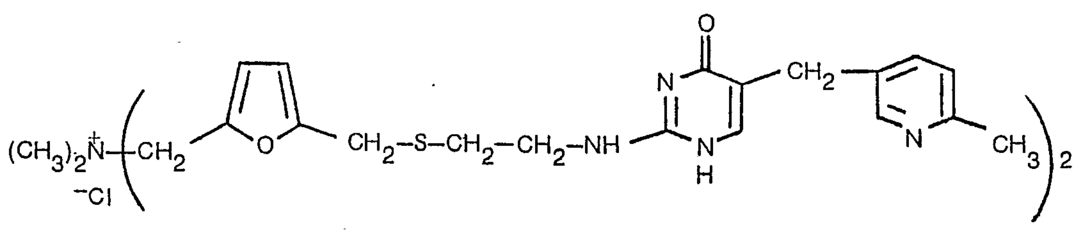 (VI)

The compound of formula (IV) as the base dissolved in dichloromethane and standing overnight forms up to 10% of the unwanted quaternary salt. This figure can be significantly reduced on the laboratory scale by speedy separation and work-up, but this is known not to be so feasible in practice on the commercial scale.

We have also found that at low pH values the compound of the formula (IV) tends to crystallise from the reaction mixture precipitating with it the starting-material of the formula (III).

We have now discovered a process that produces the compound of the formula (IV) in high yields without any significant impurity.

Accordingly, the present invention provides a process for the preparation of the compound of the formula (IV) which comprises the reaction of the dihydrochloride salt of the compound of the formula (III) with an organic solution of bis(dimethylamino)methane at a pH of about 1.3 to about 2.0.

Preferably the pH range is 1.5 to 1.6.

When used herein the pH value of the reactant solution is measured by adding an aliquot (0.5 ml) to water (5 ml) and measuring the pH of the aqueous layer.

Suitably the process is carried out in a chlorinated organic solvent, for example dichloromethane, dichloroethane, chloroform, dichloroethene or dichlorobenzene.

Preferably the bis(dimethylamino)methane is a solution in dichloromethane. It is advantageous to distil the bis(dimethylamino)methane before use.

We have found that it is convenient to achieve the desired pH of the reaction mixture by using the bis(dimethylamino)methane in an organic solution acidified to a pH of 1.3 to 2.0.

The required pH of the bis(dimethylamino)methane solution is conveniently achieved by the controlled addition of gaseous hydrogen chloride to a solution of bis(dimethylamino)methane. Alternatively, other methods of acidification may be used, for example methane sulphonic acid may be added. The acidifying agent should be one that gives rise to a counter-ion which would not cause any substantial precipitation of salt with either bis(dimethylamino)methane or the compound of formula (III).

We have found that the pH value of the reaction mixture, and therefore of the bis(dimethylamino)-methane solution, is critical for the success of this process in providing high yields and insignificant level of impurities. For example below 1.3 a major impurity is the thiol of the formula (VII) which oxidises to the disulphide

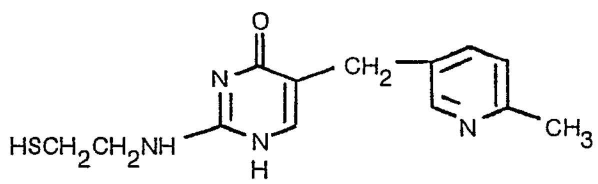

(VII)

In addition furans are known to decompose at low pH values. At pH values above 2.0 significant levels of the bis-Mannich compound are formed.

The dihydrochloride of the compound of the formula (III) is added to the acidified bis(dimethylamino)methane solution and the reaction conveniently takes place at a temperature between ambient to about 60°C, for example in dichloromethane most conveniently at ambient or at reflux, until substantially all of the dihydrochloride of the compound of the formula (III) has reacted as determined by conventional assay techniques for example by high pressure liquid chromatography.

We have found it most convenient to isolate the compound of the formula (IV) in high yields by the general process of conversion into a base, extraction into organic solvent and precipitation as the trihydrochloride or ethanedisulphonate.

The following Example illustrates the invention.

## Example 1

(i) A mixture of 1600 g (3.88 M) of 2-(furan-2-ylmethylthio)ethylamine hemisulfate and n-propanol (2800 ml) was heated to 50°C and 50% aqueous cyanamide (400 ml) added thereto. The pH was adjusted to 7.9 with 10N sodium hydroxide solution and the reaction mixture was heated to reflux. Additional 400 ml portions of aqueous cyanamide were added at 1 hour, 2 hours and 4 hours maintaining the pH at 8.3 ± 0.2 using 10 N sodium hydroxide and 6 N sulfuric acid. After 7.5 hours at reflux, the reaction was diluted with isopropanol (3800 ml and filtered whilst hot. The filtrate was diluted again with isopropanol (6000 ml) (at 40—43°) and stirred for 16 hours as it cooled. Cooling to 5—8°C gave 2-(furan-2-ylmethylthio)ethylguanidine hemisulfate (1528 g).

(ii) A mixture of 2-(furan-2-ylmethylthio)ethylguanidine hemisulfate (1190 g) and methanol (2500 ml) was stirred at room temperature. Water (43 ml) and 25% sodium methoxide in methanol (1097 ml) were added with continued stirring. Solid material was filtered off and rinsed with methanol (1500 ml). 2-(Furan-2-ylmethylthio)ethylguanidine as the free base remained in the methanol solution. Methanol (1800 ml) was distilled off, toluene (3000 ml) was added and solvent was distilled until the pot temperature was 92°C. Ethyl 2-formyl-3-(6-methyl-3-pyridinyl)propionate (646.5 g) was added and the resulting mixture was stirred under reflux for one hour. Then solvent was distilled off until the pot temperature was 110°C. Ethanol (1500 ml) was added to the hot solution. Anhydrous hydrogen chloride gas was bubbled into the solution until pH was about 1. The solution was cooled to 10°C with stirring. The solid product was filtered off, washed with 1:1 toluene-ethanol, then slurried in boiling ethanol (6000 ml). Water (55 ml) was added to dissolve the product and the mixture was stirred and cooled to 10°C. The precipitate was filtered off, washed with ethanol and dried under vacuum at 60°C to give 2-[(2-(furan-2-ylmethylthio)ethylamino]-5-(6-methylpyrid-3-yl)methyl)pyrimid-4-one dihydrochloride.

(iii) To a solution of bis(dimethylamino)methane (1 part by weight) in dichloromethane (approximately 7.06 parts by weight) was added hydrogen chloride gas until the pH reached 1.53. During this addition the temperature was kept below 15°C. 2-[2-(Furan-2-ylmethylthio)ethylamino]-5-(6-methylpyrid-3-ylmethyl)pyrimid-4-one dihydrochloride (approximately 1.38 parts by weight) was added to the mixture which was heated and maintained at reflux for one hour. High pressure liquid chromatography indicated 98.9% of 2-[2-((5-dimethylaminomethyl)furan-2-ylmethylthio)ethylamino]-5-(6-methylpyrid-3-ylmethyl)-pyrimid-4-one (the compound of the formula (IV)), 0.2% of the bis-Mannich compound of the formula (V), no starting-material of the formula (III) and no compound of the formula (VI).

**Claims**

1. A process for the preparation of 2-[2-((5-dimethylaminomethyl)furan-2-ylmethylthio)ethylamino]-5-(6-methylpyrid-3-ylmethyl)pyrimid-4-one which comprises the reaction of 2-[2-(furan-2-ylmethylthio)ethylamino]-5-(6-methylpyrid-3-ylmethyl)pyrimid-4-one dihydrochloride with an organic solution of bis(dimethylamino)methane at a pH of about 1.3 to about 2.0.

2. A process according to claim 1 wherein the pH is in the range 1.5 to 1.6.

3. A process according to either claim 1 or claim 2 wherein the bis(dimethylamino)methane is in solution in dichloromethane.

4. A process according to any of claims 1 to 3 wherein the temperature is between ambient to 60°C.

5. A process according to any of claims to 1 to 4 wherein the organic solution of bis(dimethylamino)methane is at a pH of about 1.3 to about 2.0 prior to addition of 2-[2-(furan-2-ylmethylthio)ethylamino]-5-(6-methylpyrid-3-ylmethyl)pyrimid-4-one dihydrochloride.

6. A process according to claim 5 wherein the organic solution of bis(dimethylamino)methane is at a pH of 1.5 to 1.6.

7. A process according to either claim 5 or claim 6 wherein the required pH of the bis(dimethylamino)methane solution is achieved by the addition of gaseous hydrogen chloride.

8. 2-[2-(Furan-2-ylmethylthio)ethylamino]-5-(6-methylpyrid-3-ylmethyl)pyrimid-4-one dihydrochloride.

**Patentansprüche**

1. Verfahren zur Herstellung von 2-[2-((5-Dimethylaminomethyl)-furan-2-ylmethylthio)-äthylamino]-5-(6-methylpyrid-3-ylmethyl)-pyrimid-4-on, dadurch gekennzeichnet, daß man 2-[2-(Furan-2-ylmethylthio)-äthylamino]-5-(6-methylpyrid-3-ylmethyl)-pyrimid-4-on-Dihydrochlorid mit einer organischen Lösung von bis-(Dimethylamino)-methan bei einem pH-Wert von etwa 1,3 bis etwa 2,0 umsetzt.

2. Verfahren nach Anspruch 1, in dem der pH-Wert zwischen 1,5 bis 1,6 liegt.

3. Verfahren nach Anspruch 1 oder Anspruch 2, in dem das bis-(Dimethylamino)-methan in Dichlormethan gelöst ist.

4. Verfahren nach einem der Ansprüche 1 bis 3, in dem die Temperatur zwischen Umgebungstempperatur und 60°C liegt.

5. Verfahren nach einem der Ansprüche 1 bis 4, bei dem die organische Lösung von bis-(Dimethylamino)-methan vor der Zugabe von 2-[2-(Furan-2-ylmethylthio)-äthylamino]-5-(6-methylpyrid-3-ylmethyl)-pyrimid-4-on-Dihydrochlorid einen pH-Wert von etwa 1,3 bis etwa 2,0 aufweist.

6. Verfahren nach Anspruch 5, in dem die organische Lösung von bis-(Dimethylamino)-methan einen pH-Wert von 1,5 bis 1,6 aufweist.

7. Verfahren nach einem der Ansprüche 5 oder 6, bei dem der gewünschte pH-Wert der bis-(Dimethylamino)-methan-Lösung durch Zugabe von Chlorwasserstoffgas erzielt wird.

8. 2-[2-(Furan-2-ylmethylthio)-äthylamino]-5-(6-methylpyrid-3-ylmethyl)-pyrimid-4-on-Dihydrochlorid.

**Revendications**

1. Un procédé pour la préparation de 2-[2-((5-diméthylaminométhyle)furanne-2-ylméthylthio)-éthylamino]-5-(6-méthylpyrid-3-ylméthyle)pyrimid-4-un qui comprend la mise en réaction de 2-[2-(furanne-2-ylméthylthio)éthylamino]-5-(6-méthylpyrid-3-ylméthyle)pyrimid-4-un dichlorhydrate avec une solution organique de bis(diméthylamino)méthane à un pH d'environ 1,3 à 2,0.

2. Un procédé selon la revendication 1 dans laquelle le pH est dans la gamme de 1,5 à 1,6.

3. Un procédé selon la revendication 1 ou 2 dans laquelle le bis(diméthylamino)méthane est en solution dans du dichlorométhane.

4. Un procédé selon n'importe laquelle des revendications de l à 3 dans laquelle la température est entre la température ambiante et 60°C.

5. Un procédé selon n'importe laquelle des revendications de I à 4 dans laquelle la solution organique de bis(diméthylamino)méthane est à un pH de 1,3 à 2,0 environ avant l'ajout de 2-[2-(furanne-2-ylméthylthio)éthylamino]-5-(6-méthylpyrid-3-ylméthyle)pyrimid-4-un dichlorhydrate.

6. Un procédé selon la revendication 5 dans laquelle la la solution organique de bis(diméthylamino)-méthane est à un pH entre 1,5 et 1,6.

7. Un procédé selon la revendication 5 ou 6, dans laquelle le pH voulu de la solution de bis(diméthylamino)méthane est obtenu au moyen de l'ajout d'acide chlorhydrique gazeux.

8. 2-[2-(furanne-2-ylméthylthio)éthylamino]-5-(6-méthylpyrid-3-ylméthyle)pyrimid-4-un dichlorhydrate.